# NEW EUROPEAN PATENT SPECIFICATION

(11) **EP 0 762 857 B2**
(45) Date of publication and mention of the opposition decision: **24.10.2012**
(45) Mention of the grant of the patent: 09.11.2005
(21) Application number: 96909649.4
(22) Date of filing: 20.03.1996
(51) Int. Cl.: A61F 2/78

(54) **CUSHIONING LINER**
POLSTER VORRICHTUNG
DISPOSITIFS DE MATELASSAGE

(30) Priority: 20.03.1995 US 406145; 05.03.1996 US 611306
(43) Date of publication of application: 19.03.1997
(60) Divisional application: 05076931.4
(73) Proprietor: THE OHIO WILLOW WOOD COMPANY, MOUNT Sterling, OH 43143 (US)
(72) Inventor: Arbogast, Robert E., Mt. Sterling, OH 43143 (US); Kania, Bruce G., Bozeman, MT 59715 (US); Capper, James W., Mt. Sterling, OH 43143 (US); Colvin, James M., Hilliard, OH 43026 (US)
(74) Representative: Mercer, Christopher Paul
(86) International application number: PCT/US1996/003310
(87) International publication number: WO 1996/029033

(56) References cited:
- WO-A-95/27756
- WO-A1-95/05792
- WO-A1-95/31160
- SU-A- 1 812 982
- US-A- 4 502 234
- US-A- 4 542 169
- US-A- 4 635 626
- US-A- 4 814 375
- US-A- 4 822 371
- US-A- 4 908 037
- US-A- 4 923 474
- US-A- 5 262 468
- US-A- 5 334 646
- US-A- 5 336 708
- US-A- 5 376 132
- US-A- 5 443 525
- O. KRISTINSSON: 'The Iceross concept: a discussion of a philosophy' PROTHETICS AND ORTHODONTICS INT. no. 17, 1993, pages 49 - 55
- ' Kraton Thermoplastic rubbers in oil gels' TECHNICAL BULLETIN SHELL CHEMICAL COMPANY vol. 1102, no. 89, April 1989, pages 3 - 10
- 'Otto Bock Gel-Strumpf "Derma Seal"' ORTHOPÄDIE TECHNIK vol. 11, November 1993 - November 1993, pages 1 - 4

## Description

The present invention relates to a cushion liner for enclosing an amputation stump, preferably for use by, e.g., below-knee (BK) amputees. The cushion liner is preferably provided in a contoured form fit configuration which adapts to a right or left side bias of the bony prominence of the residuum (stump) or is provided in simple tube (i.e., tube-sock) shape with various optional cushioning. Cushioning material is provided on the inside and may optionally be provided on the outside of the liners to minimize the discomfort of, e.g. an orthotic device, such as a knee brace, or a prosthethic device, such as an artificial arm or leg. In a preferred embodiment, the cushioning material is adjusted in thickness and has a non-uniform thickness over the article surface. In another preferred embodiment the liner has cushioning material in a recessed achilles configuration: the cushioning material does not contact the wearer at an upper posterior (i.e., knee crease), or upper anterior (i.e., elbow crease, etc.) portion of the limb or residuum, or minimally contacts the wearer at these positiond due, e.g., to the thinning of cushioning material. For example, the cushioning material can be thinner in these areas than in other places.

### Discussion of the Background:

For at least the past 80 years amputees have worn tubular socks over their residual limb. Cotton, wool and cotton-wool blends have typically been used. More recently with the advent of synthetic materials, nylon and other textiles, including some with a measure of elasticity, have also been utlized.

In a typical below-knee (EK) prosthesis an amputee's stump tends to "piston" in the socket: during ambulation the stump will come up in the socket of the prosthesis until the attaching means holding the prosthesis to the wearer cause the prosthesis to lift with the stump. On the way down, air may be trapped between the residuum and stump sock, or between the prosthesis socket and sock, or between a socket liner and a sock.

With wool and cotton socks which tend to breathe and which are not airtight this pistoning effect is not a major problem with regard to the generation of sound effects. Since wool and cotton tend not to tightly form fit a residuum, however, the amputee typically packs a material around the residuum once it is placed into the prosthetic device or adds additional socks to increase thickness or puts on thicker socks in order to provide necessary fit. However, for socks which do not breathe and which are made from, e.g., polymeric material, a problem occurs when the residuum pistons in the prosthetic device: terrific sound effects such as sucking and gurgling noises are generated which are obtrusive and inappropriate, often embarrassing the wearer. In addition, such air pockets produce non-uniform pressures and loading discontinuties on the skin, irritating it.

Finally, many amputees experience a swelling of the stump. When the residuum is in a prosthetic socket the stump tends to contract significantly, and when taken out of the socket the stump tends to expand within minutes of removal. This expansion and contraction of the residuum contributes to the development of air pockets and the generation of obtrusive noises since a sock which may have provided a comfortable fit on the expanded stump becomes a loose fit with air pocket opportunities when the residuum is placed inside the prosthetic socket. In addition, and over time, an amputee's residuum tends to adjust in size, usually shrinking. As these changes occur they increase the tendency for the pistoning effect, described above, to occur. In addition to the embarrassment caused by the sound effects generated by pistoning, cushioned socks which allow or promole air pocket formation quickly wear out and, if not replaced often, lead to lesions, etc. on the residuum.

Currently available cushioned residuum socks are tubular or conical and do not provide a form fit on an amputee's residuum. Regardless whether such socks are provided with internal and/or extermal cushioning material they fail to avoid air pockets. While a stump may generally have a roughly conical or cubical shape there are invariably recessed areas on, e.g., the medial side of the prominent tibia bone. Generally, on a below knee, left side residual limb the recessed area will be predominantly on the right side of the tibia bone. There is also typically a smaller recessed area on the left side. For right side residual limbs the predominant recessed area is on the left side of the bone, with smaller recessed areas on the right side. Usually the greatest recess occurs immediately below the patella, on either side. In addition, left side amputees typically have a right side bias to the bony prominence of the below knee stump, and right side amputees have a similar bias to the left side. Conventional tubular or conical elastic socks simply cannot account for those several variable conditions without using extremely high levels of elastic tension which compress the outer-most points along the stump's circumference, causing discomfort and a non-uniform fit.

Amputees typically attach a prosthetic limb to their residual limb by means of a rigid socket, liner, and a suspension means. The rigid socket is often custom fabricated to match the shape of the intended user's resuidual limb and may be made of thermoplastic or fiber-reinforced thermoset materials, but can also be made from wood, metal, etc. Since such hard materials are generally uncomfortable when in intimate contact with the skin over long periods of time, especially under load bearing conditions, liners and/or prosthetic socks are often used as interface members between the hard socket and the residual limb to increase comfort. Such liners are generally of the open cell foam type, such as Pelite or Kemblo, but may also be made of silicon, urethane, etc. type materials. See, for example, U.S. 5,258,037 and U.S 5,378,152. Prosthetic socks, as mentioned above, may be made of wool, cotton, synthetic materials, etc, and amputsee tend to prefer liners and socks which are easily changed to facilitate cleaning, to accommodate volume changes in the residual limb, or to accommodate different user activities.

US 4,635,626 (LERMAN) discloses a stump sock for amputees comprising a flexible resilient material including a base layer of a flexible resilient open cell material; a skin-protecting firet layer of a soft flexible poroous material overlying a first face of the base layer, and a second layer of a protective fabric overlying a second face of the base layer

Suspension sytems which help, to hold a prosthetic limb in place may or may not be an integral part of the rigid socket and/or liner. Examples of suspension systems include supracondylar or walet belt, joint and corset systems, neoprene or latex sleeves, socket ears which grip the condyles, suction or pin and lock systems such as those where the pin is attached to a liner and the lock is attached to hard socket, etc. Examples of typical suspension systems may be found in U.S. 4,923,474, U.S. 4,923,475, U.S. 5,007,937, U.S. 5,108,456, U.S. 5,201,773, U.S. 5,201,774, U.S. 5,246,464, U.S. 5,263,923, U.S. 5,314,497, U.S 5,387,245, U.S. 5,376,131 and U.S. 5,405,405.

However, and as is clear from the above description of the prior art, all current interfaces for use between an amputee's residual limb and a prosthetic device sulfer from drawbacks which may include custom fabrication (and corresponding long lead times), high cost, low durabllity space requirements (too long, too high profile, etc.), noise due, for example, to air pockets forming between the liner and the residual limb, skin irritation, restricted joint range of motion, lack of accommodation of stump geometry changes, objectionable odors, discoloration, inadequate comfort, etc.

### SUMMARY OF THE INVENTION

The present invention provides a cushion liner as defined in claim 1. Preferred features of the cushion liner of the present invention are defined in the dependent claims.

### BRIEF DESCRIPTION OF THE DRAWINGS

A more complete appreciation of the invention and many of the altendant advantages thereof will be readly obtained as the same becomes better understood by reference to the following detailed description when considered in connection with the accompanying drawings, wherein:
Figure 1 shows a typical pattern for the reflected two-piece form fitting sleeve member according to the invention.

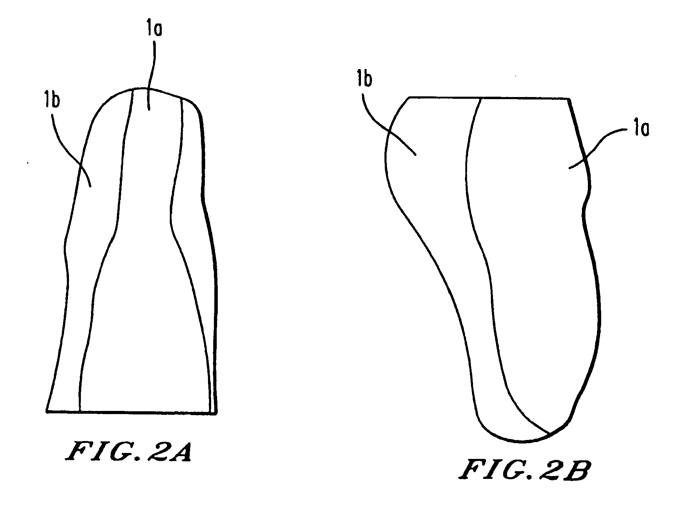

Figure 2 shows frontal (A) and side views (B) of the invention sleeve member enclosing a stump-like form, where 1a and 1b rater to pattern members a and b, respectively, in Figure 1.
Figure 3 shows a typical pattern for the optionally banded three-piece form fitting sleeve member according to the invention, piece (a) being optional on the Figure 3 pattern. Piece (a) can also be used in the Figure 1 pattern to provide a top band.

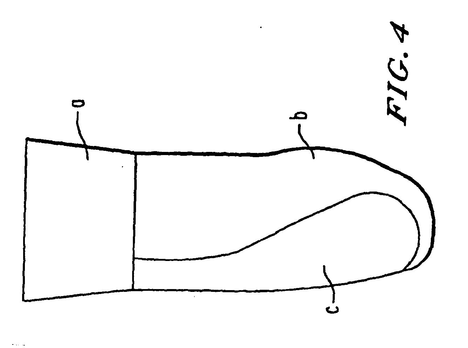

Figure 4 shows an invention sleeve member assembled from the Figure 3 pattern, where a, b and a compound to patterns a, b and c, respectively, in Figure 3.

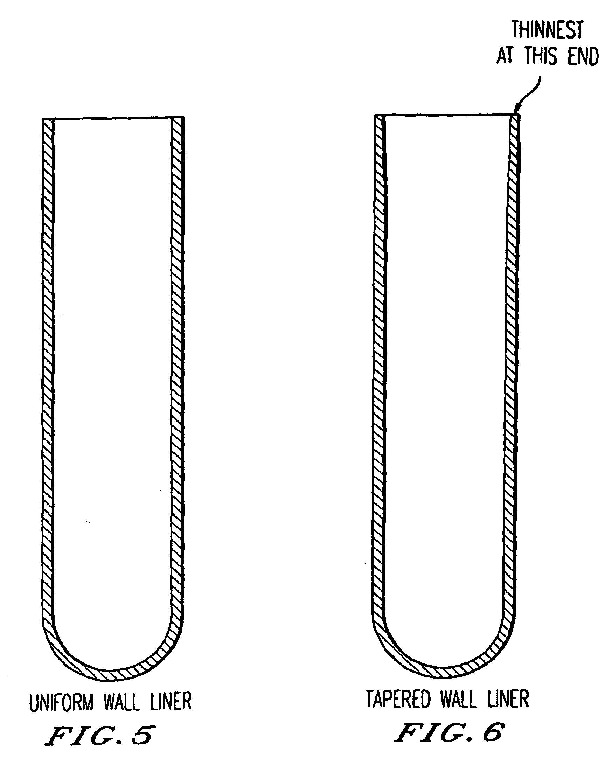

Figure 5 shows a cushion liner according to the present invention with uniform wall thickness.
Figure 6 shows a cushion liner according to the invention having a tapered wall thickness at the open end.
Figure 7 shows side and front views of an invention cushion that which has a contoured inner surface providing Variable thichness cushioning material at portions of the inner intended to provided particular selective cushioning to the user.

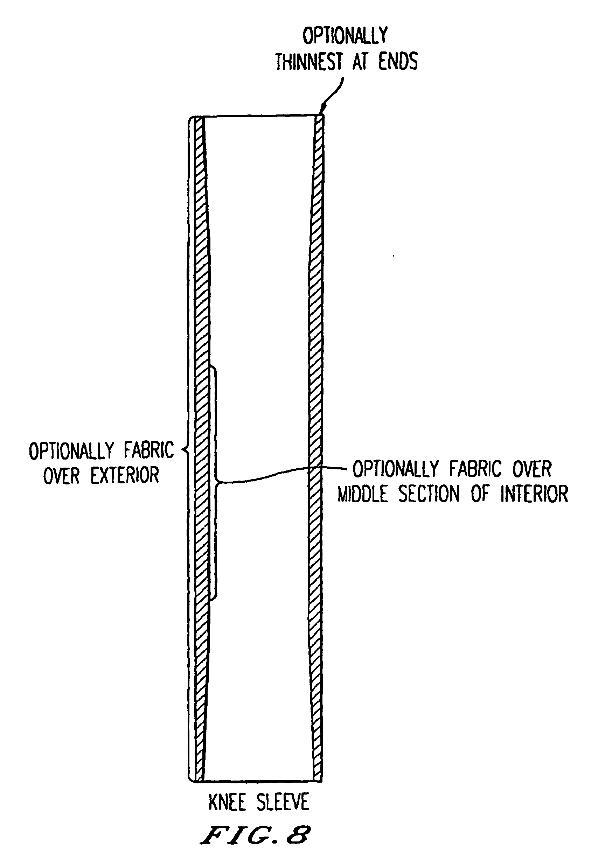

Figure 8 shows an open-ended cushion knee or elbow sleeve with optional fabric covering and with optional thinning at both ends.

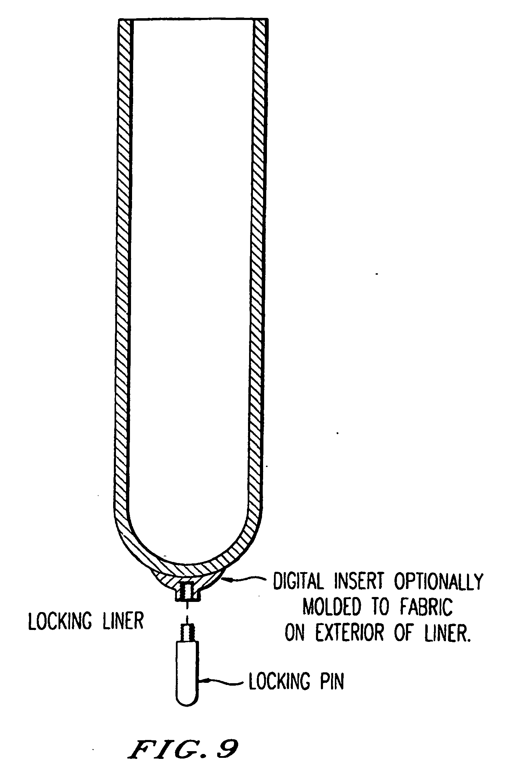

Figure 9 shows an invention locking liner with docking means at the distal end thereof.

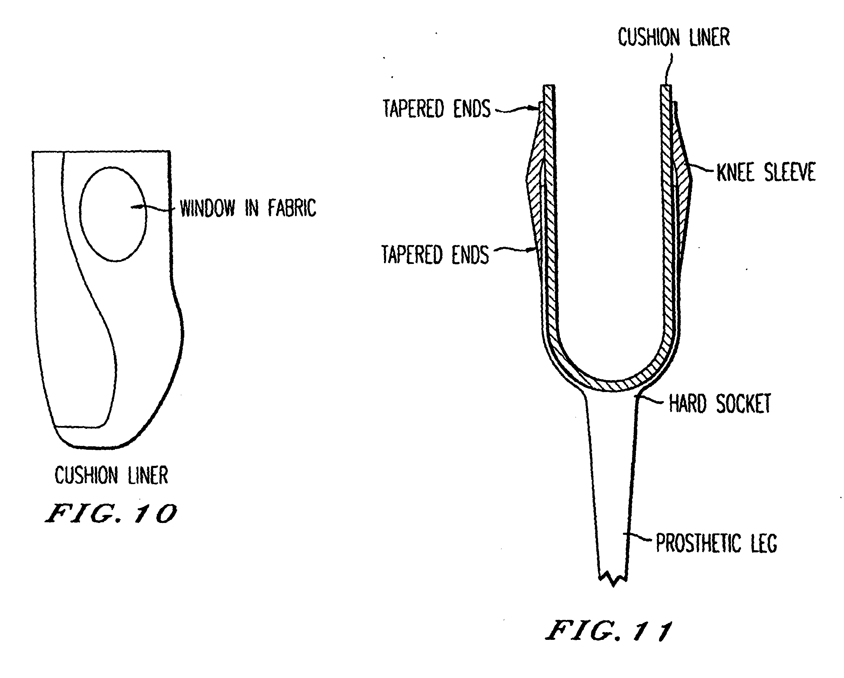

Figure 10 shows an invention form fitting sleeve having an optional window of clear plastic material, etc., in the fabric.
Figure 11 shows an invention open-ended knee or elbow sleeve in position and contacting a cushion liner and a prosthetic device.

### DETAILED DESCRIPTION OF THE PREFERRED EMBODIMENTS

A polymeric gel composition useful in the present invention comprises, preferably, a block copolymer and mineral oil. The gels are nonfoamed or foamed with, e.g., a foaming agent. The mineral oil may be present in from 5-85% by weight based on total weight, more preferably 20-50% by weight, but also including all of any positive amount including 10, 15, 20, 25, 30, 35, 40, 45, 55, 60, 65, 70, 75 and 80% by weight and all values and ranges in between all these listed values. The gel preferably has a durometer (Shore A) of 1 - 20 and preferably a durometer that matches or approximates (±10%) human skin. Preferably, the oil is present on an equal weight basis, or in a weight ratio of 1/4, with regard to the amount of polymeric material present. The polymeric material present is preferably a styrene isoprone/butadiene block copolymer or styrene-ethylene/butadiene-styrene block copolymer. Preferable examples of such polymeric materials useful herein include C-Flex 1970-W5 (R70-338-000). C.Flex 1960-W5 (both manufactured by Consolidated Polymer Technologies, Largo, Florida, U. S.A) and Kraton G1654 (manufactured by Shall Chemical Co.). Forthe C-Flax materials a particularly prefered ratio is 1 part oil per 2 parts C-Flex material.

Preferred ratios of polymer to mineral oil are 1/1-4/1 using C-Flex 1970-W5 or 1980-W5, one part Kraton G1654: 2.75 parts mineral oil, and 14 parts Kraton G1654; 16 parts C-Flex R70-306 (or R70-190 or R70-251 or any mixture thereof): 40 parts mineral oil The C-Flex R70-339-000, R70-306, -190 and -251 materials are also preferred herein and are products of Consolidated Polymer Technologies. They are blends of S-EB-S block copolymer or SIB block copolymer with mineral oil, 10 parts Kraton G1654 and 11 parts C-Flex R70-306 and 27 parts Duoprine 70 oil is also preferred.

The preferred polymers useful herein and listed above (C-Flex and Kraton materials), in addition to being styrene-isoprone/butadiene or styrene-ethylene/butadiene-styrene block copolymers (mixed with mineral oil in the case of the C-Flex R70-339-000, R70-306, -190 and -251 materials) also include styrene-butadiene-styrene and any thermoplastic elastomer having the Shore A characteristics listed above and capable of being blended with mineral oil. Mixtures of all mentioned polymers may be used. Several preferred polymeric materials useful in the present invention are more particularly described with regard to the invention sleeve member infra.

The mineral oil used herein is preferably purified mineral oil and is preferably USP grade.

The present invention cushion liner may have an overall tube-sock shape or may be form-fitting (described more fully below with regard to the invention sleeve member). These shapes are referred to generically as sock-shaped coverings. The invention sleeve cushion liner can fil a range of residual or normal limb sizes with minimal or no air pockets, and preferably have a range of elasticily of from 10-400% and a range of distal radius of 19-102mm (3/4"-4") or whatever is required by the wearer. The cushion liner may be made of a combination of gel/fabric with appropriate seaming, where necessary. At least three standard geometries may be provided for the invention cushion liner, those geometries being 1) uniform wall. 2) tapered wall and 3) contoured wall. These geometries are also useful with regard to the invention sleeve and open-ended sleeve member discussed below and refer to the thickness of the gel. Recessed achilles configuration (use infra) can be used in all aspects of the invention.

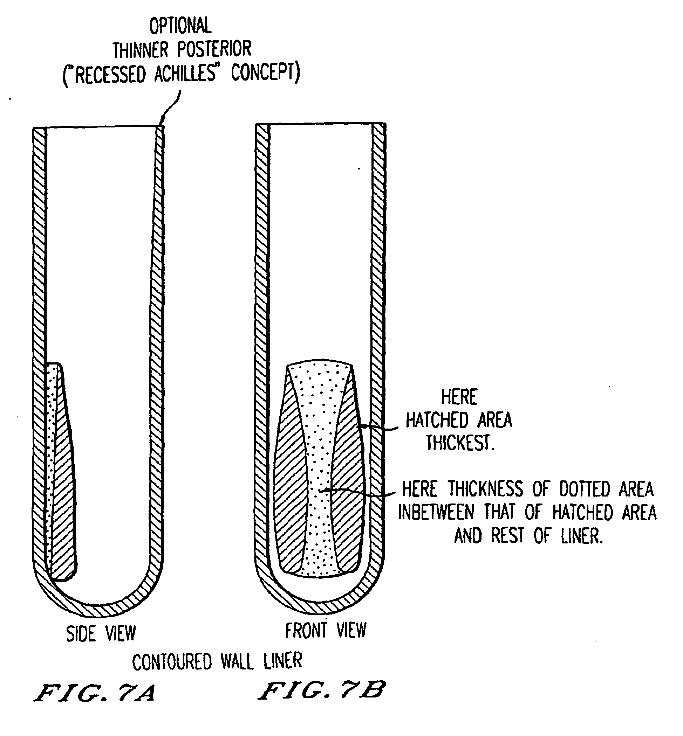

The uniform wall cushion liner simply comprises a uniform thickness of gel. Tapered wall cushion liners are generally those having a layer of gel which is thicker distally for additional padding (and because most shrinkage of the residual limb occurs at this point of the limb) and thinner proximally (near the open and of the liner) to blend in and interface more easily with the residual limb. Contoured wall cushion liners have uneven distribution of gel throughout to provide cushioning effects where needed and, in a preferable embodiment, have a thinner posterior middle and upper to allow maximum range of motion optionally with a thicker distal end both anterior-medial and anterior-lateral with less thickness in the region between these two areas so as to pad typical bony promenances. Contoured wall liners are often thicker distally and custom shapes can easily be provided to satisfy the individual user. For example, in the liner of Figure 7, the hatched area has a gel or polymeric material cushion thickness of 13 mm, the dolted portion 11 mm, the front of the liner 9 mm and the portion for behind the knee less then 9 mm.

For the invention cushion liner combinations of gel with fabric include gel with a two-piece or three-piece form-fit sleeve (described below). Other configurations include gel coating inside a tube-sock fabric form.

Foamed or nonfoamed thermoplastic elastomers or rubber only can also be used as cushioning material alone or in combination with the gel in the invention. The term "thermoplastic elastomers" has its typical meaning and excludes the gel referred to above. The foamed materials can exclude mineral oil. The inclusion of thermoplastic elastomers in the gel (the mixture optionally being foamed) is advantageous in making the products customizable since such products will tend to take the shape of a limb or model of a limb when, e.g., heat and/of pressure are applied, in a preferred embodment the gel can be foamed and used in combination with fabric in all forms of the invention.

In addition, in the cushion liner, transducers can be included therein to sense pressure, force, temperature, etc., to detect and/or transmit a signal from the residual limb to a prosthetic device, to send myoelectric signals, etc. In addition to transducers, any electrical device or other sensing device can be similarly incorporated for detection, signal transduction, etc. See, for example, U.S. 5,443,525.

The cushion liner may comprise docking means for attaching an external device, etc. to the liner. Such docking means includes pins, etc. and are typically those which help to attach and support a prosthetic device. These docking means are known in the art and are preferably incorporated in the cushion liner by means of direct molding, meaning the injection molding of an adapter into the fabric, etc. Such docking means, including distal inserts, can be centered or can be offset to accommodate individual residual limb geometries. Other docking means include molding a raised configuration in the side of the liner which then mates with a recess on the inside of the prosthetic socket, allowing for a loocking affect when the user dons the liner and steps into a socket.

An open-ended cushion knee or elbow sleeve (also referred to herein as cushion knee sleeve or knee sleeve, etc., for brevity) is intended to be worn by an amputee and provide an interface between the residual limb and a prosthetic device, and is worn external to both, or may be worn by a person whose limb is intact but desires or requires padding or joint support. The knee sleeve is generally cylinder- or band-shaped and covered on the exterior with fabric and coated on the inside with invention gel. The sleeve can be any size but typically is from 25.4-635 mm (1-25 inches) long including 254, 381, 406.4, and 508 mm (10, 15, 16, and 20 inches), and any diameter (unstretched) such as 25.4 - 254 mm (1-10 inches), including 50.8. 66.9, 101.6 and 127 mm (2, 3½, 4 and 5 inches), Fabric may cover the middle section of the interior, it desired. The cushion knee sleeve itself may have a conical (i.e., tapered) shape with a smaller diameter distally than proximally or smaller diameter distally and proximally as compared to a central diameter so as to grip and hold the prosthetic device or residium at the smaller diameter end(s). The interior gel coating can be thinner at either or both of the distal and proximal end, and can be thinner or absent in the back of or whole of the middle section thereof so as to not bind in the crease of the knee or elbow when worn by the user. It is preferred that the wall thickness of the gel be thin at the ends regardless whether there is fabric covering the gel or whether the exterior or whole cushion knee sleeve is made simply of gel itself.

The cushion knee sleeve can be used in combination with the invention cushion liner as a means for suspension of a prosthetic device, or can be used alone. In addition, the knee sleeve can have attached thereto, by molding into the gel, by attachment means such as pins, etc., an orthotic knee joint and optional support bars such that the sleeve constitutes a knee brace.

All articles such as the (closed-ended) sleeve and liners can be provided with gel or thermoplastic on the outside thereof so as to come in contact and provide increased friction with the interior gel of the cushion knee sleeve. Such a configuration provides additional support and suspension of the prosthetic device.

The present invention liner for enclosing an amputation stump overcomes the problems encountered with prior art tubular or conical socks which are either prone to air pocket sound effects or are so constricted as to be uncomfortable by providing a sleeve member which is made in the shape of or from a pattern and comprises the gel optionally combined with elastomer which provides elastic tension such that the liner form tilts an amputee's residuum.

This provides a sleeve member having a comfortable feel and avoiding the generation of obtrusive sounds which are directly traceable to the presence of air pockets between a sleeve member and an amputee's residuum or between sleeve member and prosthetic socket.

In all aspects of the invention described herein, the elasticity of the liner is preferably sufficient to accommodate the swelling or shrinkage of the residual limb typically experienced by an amputee and still maintain an intimate fit. The liner of the invention has enough elastic compression to form fit a stump but is not so tight as to be considered a stump shrinker, as in U.S. 4,840,635.

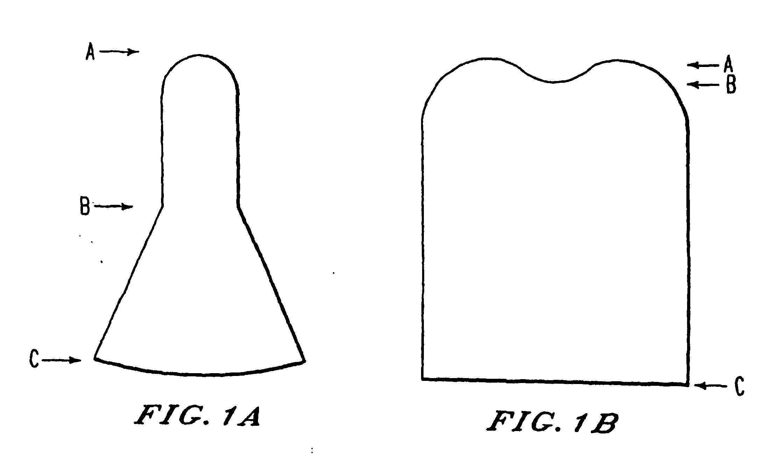

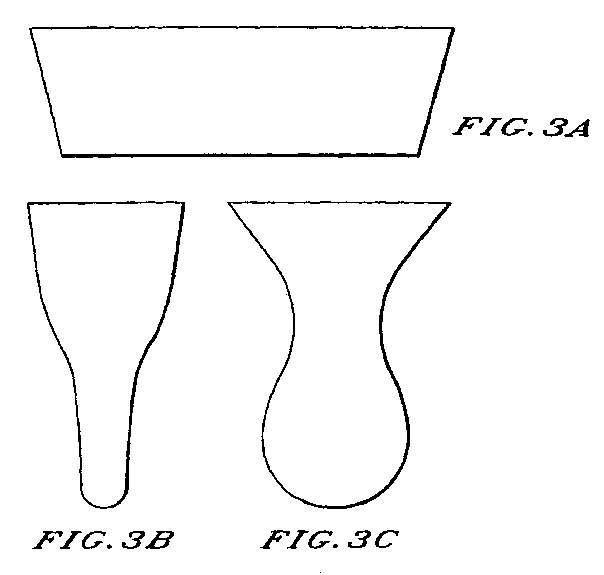

Figure 1 depicts a typical pattern from which the present invention form-fitting sleeve member is constructed or shaped into. The pattern is a reflected two-piece pattern, one piece of which is designed to cover the bony prominence of a typical BK stump, (Figure 1a) the other piece joined to the first at the edges thereof and circumscribing the typical onset of soft tissus around the stump (Figure 1b). The two patterns can be used to cut out two or more pieces of textile material which are brought together such that the "X" on each of the patterns in Figures 1a and 1b are in contact with the "X" on the ether pattern, followed by the sewing together of the edges of each pattern in typical fashion. When the two pieces are sewed together, a sleeve member is provided which has a form fitting residuum-like (tubular) shape having an open and into which an amputation stump may be introduced, a closed end opposite to the open end, an interior and an exterior. The two-piece pattern may be cut out of the same textile material or different textile materials, and the two pieces of textile material may have the same color or different colors. The three-piece optionally banded reflected pattern of Figure 3 also provides a form-rit sleeve, piece (a) being optional.

The form fit sleeve d the invention can be made from fabric, with gel, elastomer, end combinations thereof according to the patterns in Figure 1 or Figure 3. In Figure 1a the distance A-B divided by the distance B-C generally varies from 2/1 to 1/2 end is preferably about 1/1. The width of the pattern in Figure 1a at point B divided by the width at point C le generally approximately from 1/4-1/1, preferably about 1/2. In Figure 1b the distance A-C divided by the distance B-C is generally preferably about 1.05-1.3, most preferably about 1.1. In both patterns of Figures 1 and 3 the dimensions may be varied so as to provide a comfortable form fit that avoids air pockets.

The two or more pieces of textile material used to form the invention form fitting liner can be sewn together using any type of thread and any type stitch. This is also true for tube-sook shaped articles. In a preferred embodiment, woolly nylon is used to interconnect the two-piece or three-piece form-fitting liner of the invention or seem the tube-sock using a flat-locked stitch, which is a stitch well known to those in the art. This flat-locked slitch (ends to create a smooth, non-irritating seem having e stretch comparable to jersey fabric.

The size of the liner according to the invention can be varied depending upon the residuum to be enclosed by simply proportionally reducing or enlarging the pattern, as desired. The term "form fitting" residuum-like (tubular) shape as used herein refers to the shape of the invention liner which provides a contoured fit on an amputation stump, which substantially reduces or eliminates air pockets during pistoning of the amputation stump in a prosthetic socket and which is obtained by providing a liner composed of two or more pieces of fabric having the pattern described in Figure 1 or Figure 3 and/or comprised of gel and/or other elastomeric material in the shape provided by these patterns. Residuum-like configuration is further achieved via a bias molding technique that replicates contours of a normal amputation stump.

The fabric-containing articles according to the present invention may be made with any textile material having any thickness (ply). Preferred textile fabrics are those having elasticity, including elasticities of 10-400%, such as stretchable non-wovens (e.g., the Xymid® line of fabrics including Wearforce® fabrics from DuPont which connect bulkable yarns with non-woven sheet substrates), Lycra® comprising segmented elastomeric polyurethane fibers (spandex), supplex nylon (an engineered nylon textile fabric with a cotton-like texture end appearance), neoprene fabrics (polychloroprene fabrics), nylon, spunbonded olefin, looped nylon, spunlaced fabrics, polyester, around fiber fabrics, etc, However, any textile material may be used such as those described in Textile, fourth edition, N. Hollen et al, Mac-Milan, New York, 1979, The Modern Textile Dictionary, Duell, Sloan and Pearce, New York, 1963 and Dyeing Chemical Technology of Textile Fibers, Trotman, E., Charles Griffin and Co., London, 1975: The fabrics used to make the invention articles are preferably elastic and are preferably jersey knit but include all woven, knitted and non-woven textile fabrics, in addition to those mentioned above and described in the above-mentioned references, those described in Volume 22, p. 762 ff and Vol. 16, p. 72 ff of the Kirk-Othmer Encyclopedia of Chemical Technology, Wiley, New York, 1983 and 1991, respectively, are also included.

Mixtures of types can be used with asaming where necessary.

Preferred fabrics include mixtures of the above-mentioned fabrics, such as a fabric of neoprene, 88% supplex nylon/12% lycra spandex, 85% nylon/15% lycra spendex, 94% polyester/6% lycra spandax. Such mixed fabrics may be uniformly mixed or may have one type of fiber or predominantly one type of fiber on one face thereof. For example, in those fabrics described above which contain lycra, the lycra can be mixed throughout, can make up the entire or substantially the entire face, or the entire or substantially the entire back of the fabric once it is arranged in an invention article.

The textile fabrics used in the invention may be treated/finished in any manner known in the art. For example, a nylon tricot surface may be applied to the textile fabric, etc. The finishing need not be uniform over the entire invention article. The article may be selectively treated at, for example, above the knee (or elbow) portions, and with the same treatment, no treatment or another treatment being present below the knee or elbow. Similarly, treatment on the outer surface of the invention sleeve member may be different from that on the inside thereof.

The textile material used to make the invention liners is preferably elastic (stretchable) in one or more, preferably two, directions and is capable of adjusting to variations in form and size of the residuum or limb. In a preferred embodiment, a nylon, neoprene, looped nylon combination provides excellent comfort and durability. The thickness of the textile material range from 0.635 to 3.175 mm (.026in to .125in), all values and all ranges therebetween. Typically the thickness of patterns pieces in Figures 1 and 3 are the same, but need not be.

The liner according to the invention is preferably a cushioned sleeve member, that is a sleeve member having a form fitting shape with an open and into which an amputation stump may be introduced, a closed end opposite to said open end, an interior and an exterior, wherein the interior at the closed end is impregnated with a polymeric material arranged so as to provide a cushion between the amputee's residuum and any prosthetic device to be worn, attached to, etc. the residuum. The cushioning material is preferably a polymeric material, most preferably the aforementioned gel and/or a thermoplastic elastomer (referred to simply as thermoplastic herein) such as a thermoplastic rubber, silicon containing elastomer, etc. which provides an interface between the residuum and a prosthetic device but which does not contact or minimally contacts the skin at the back of the knee or elbow when worn by an amputee (recessed achilles). This cushioning material may also here and in other articles of the invention be a thermoset silicone. This cushioning material is thus provided in a "recessed achilles" arrangement which avoids the irritation occuring in the crease behind a knee or elbow provided by prior art cushion sleeves by thinning or eliminating cushioning polymeric material at this location, If the form fit sleeve is to be worn by an above-knee amputee the recessed (thinned or absent) portion of the cushion material may be arranged medially at, e.g., the perineum for increased comfort.

The polymeric material which provides the cushioning effect in all aspects of this invention may be any polymeric material. Preferred materials other than the gel and those described above are those elastomers described at pgs. 446-640 of volume 8 of the Kirk-Othmer Encyclopedia of Chemical Technology, Wiley, New York, 1979 and those rubbers described in Synthetic Rubbers. Their Chemistry and Technology, Blackley, D., Applied Science Publishers, London,1983 and Rubber Technology, Morton, M. Ed., Van Nostrand Re-inhold Co., New York, 1987.

A preferred embodiment of the present invention liner includes a cushioning material of Kratone®-type rubber material including those obtained from Shell, CPT, and GLS. These Kraton® rubbers are styrene-ethylene/butylene-styrene block copolymers or styreneethylene/propylene block copolymers or styrene isoprene/butadiene block copolymers and are available in triblock or diblock form. See, e.g. the Kraton® Technical Bulletin from Shell Chemical Company, SC:1102-89, June, 1992.

The cushioning polymeric material used in the present invention cushioned sleeve member is characterized by a certain durometer range. Durometers for the invention cushioning material preferably range from 1-20 on the shore "A" scate. The lower the number the softer the material, typically due to a higher level of plasticizer. One preferred durometer range is 3-14 including all values therebetween and all ranges therebetween.

The polymeric cushioning material may be a blend of, e.g., Kraton® rubbers and oils such as mineral oil, etc. Including typical stabilizers, etc. which provide an average durometer of from 1-20, preferably 3-14. These blends typically comprise a rubber having a lower durometer (1-10 on the shore "A" scale) and a rubber having a higher durometer (e.g., 11-20). The blends are preferably capable of being stretched 100% or more, preferably 400% or more before tearing and are capable of providing a form to the residuum due to their inherent elasticity. In addition, low durometer Kraton® rubbers and other material tend to have a sticky feeling which, when present in the polymeric cushioning material, tends to enhance the form fitability or the sleeve essentially by mating against the skin.

In donning those liners of the invention which, when worn, provide contact between, e.g., the gel, a thermoplastic material, a combination thereof, etc., with the skin and/or a prosthetic device it is preferred that the invention liners be donned in a manner such that the polymeric material does not drag against the skin, For example, the invention cushion liner or sleeve can be rolled befors donning, and then unrolled on the limb and/or device. In this manner, the cushioning polymeric material encloses the limb and/or device without sliding or friction. If the liner has an outer textile surface, the textile material slides against itself, providing easy action. With regard to the open-ended sleeve described herein a particular advantage is obtained when this open-ended sleeve has an interior middle band of fabric. The distal and proximal portions of the open-ended sleeve can be rolled towards the middle of the sleeve, and the sleeve can be donned with contact between the wearer or device and fabric only. The thermoplastic-containing portions can then be unrolled onto the wearer and/or device. In all cases, the invention article can be taken off by reversing the above-describe processes. This aspect of the invention (assay donning and doffing) is an important advance in the art provided by the present invention. No lubricant, talcum powder, etc. is required, as with currently available materials, in addition, the invention articles regardless of their composition, can be adapted such that the portion thereof which will come in contact with the user's appareil such as pant legs, shirt sleeves, etc. is fabric or covered with fabric such that the wearer's apparel does not stick, to end bunch on the invention articles.

If desired, the cushioning material may comprise antioxidants such as Vitamins A, B and C or any other antioxidants commonly used in polymers which can weep out on a time release basis. In addition, skin conditioning agents may be added to the polymeric material to soothe the skin during wear. Such skin conditioners include mineral oil, baby oil, etc, which may be added to the polymeric material prior to its application to the sleeve member. Also, astringents, biocides, medicaments, etc, may be added or applied to the cushioning material to avoid infection or heal sores, etc.

As described above, the cushioning material is preferably formed in a recessed achilles fashion on the interior of the invention articles. Cushioning material may also be applied to the exterior. In both cases, it is preferred that the cushioning material be applied such that it provides an interface between the amputee's slump and a prosthetic device or provides padding and/or joint support but minimizes or eliminates contact with the skin at the back of the knee or elbow when worn. The cushioning material may be separated from the skin by a piece of fabric, by an interior sock liner, or may contact the skin directly. Such contact with the skin can reduce sweating, etc.

While several methods may be used to apply the cushioning material to fabric, a preferred method includes the dipping of the closed end of the article into molten or liquified cushioning material at an angle of from 15° to 80°, preferably 20-50°, most preferably 24-45°, with respect to the surface of the molten or liquid cushioning material. In this manner, the cushioning material extends up the article from the closed end thereof to a further extent on the side of the liner, sleeve, etc. to be positioned in from or the knee than behind the knee (e.g., the pattern in Figure 1a faces forward on a BK amputee). As long as the cushioning polymeric material minimizes or eliminates contact with the skin at the back of the knee or elbow when worn but still provides an interface between the amputee's stump and a prosthetic device or provides join support and/or padding, the material is in a recessed achilles configuration. Preferably the polymeric material comes up at least about 12.7. 457.2 (½-18), preferably 12.7-254 (½-10), more preferably 76.2 - 203.2 mm (3-8 inches), including all values and ranges therebetween these several values, from the closed end at the articles in front of the knee or elbow and covers the knee or albow. The difference in neight of the cushioning material behind (i.e., in the crease of) the knee or elbow as opposed to in front of the knee or elbow can differ by several tens of mm (inches) measured from the closed end of the article, typically from 25.4 - 381 mm (1-15 inches), preferably 25.4-203.2 (1-8) and includes all values there between and all ranges therebetween these several values. In a preferred embodiment the cushioning material is thicker at the closed end of the article than it is towards the open end.

In addition to the application of the gel and/or polymeric cushioning material to, e.g., the sleeve member by dipping into liquified or molten polymeric material or painting the material on the article, etc., it is possible to dissolve the polymeric material in a solvent followed by application of the solvent to the article with subsequent evaporation of the solvent. Close control of the thickness of the polymeric material is obtained using this method. In both the direct dipping and solvent methods the articles is generally spun with distal end angling dowrward to provide tapered thickness while drying. In general, the thickness of the polymeric material applied to the articles in any fashion including in a recessed achilles fashion can be any thickness to, e.g., several tens of mm (inches) but preferably varies from 0.0254 - 127 mm (.001 -.500 inches), preferably 0.279- 3.81 mm (.011 .150 inches) but all values and all ranges therebetween these several values, end can be substantially nonconstant in thickness throughout. For example, the cushioning material preferably may be thicker at the closed end of the sleeve (e.g. 3.175 (.125in)thick) and be tapered or feathered in decressing thickness as the open end is approached. Such changes in thickness can be accomplished by techniques known to those of ordinary skill in the art and are within their skill. For example, compression molding can be used.

Another preferred method of producing the invention articles injection molding. The article is pulled over a core and inserted into a cavity with polymeric material being injected into the cavity.

Other features of the invention will become apparent in the course of the following descriptions of exemplary embodiments which are given for illustration of the invention only and are not intended to be limiting thereof.

### EXAMPLES

### Example 1

A form-fit next-to-skin sock was prepared from an 88% supplex nylon/12% lycra spandex jersey knit fabric using woolly nylon thread and a surged flat-locked stitch. The sock comprises two pieces of fabric, the first piece having the pattern described in Figure 1a, the second piece having the pattern described in Figure 1b.

A mixture of melted Kraton® rubbers obtained from Shell (G1652) and GLS (6705) and Duoprime® 70 oll (mineral oil) was prepared, the sewn inverted sock was then placed over a mold facsimile of an amputation stump having recessed portions at what would be either side of the tibia and dipped into the molten Kreton® blend at an angle of 24°-28° with regard to the plane of the surface of the molten Kraton® and removed. The mold was spun during drying. A form-fit cushioned stump sock was obtained having adhered cushioning material in a recessed achilles arrangement on the interior thereof.

### Example 2

A 1.5785 mm (1/16 inch) thick neoprene textile fabric with nylon tricot surface treatment for above the knee contact was used to prepare a three-piece form fitting sleeve member according to the present invention using the pattern described in Figure 3. The 1.5785 mm-(1/16 inch)neoprene material for the below the knee segment of the invention sleeve had nylon on the exterior side and looped nylon on the interior side. The against the skin side of the above knee segment of the invention sleeve was neoprene which provided a high friction bond. This form fitting sock was dipped into molten Kraton® (a blend of tough end soft Kraton® used in Example 1) at an angle of 24°-28° to provide a cushion material on the interior thereof. The resultant composite sock of nylon, neoprene, looped nylon and cushioning rubber provides e durable cushioned sleeve member which, when impregnated with rubber, has an approximate thickness of 3.175 mm (⅛ inch.

### Example 3

A commercial cotton tube sock is inverted and dipped into molten elastomer at an angle of 26° relative to the plane of the molten elastomer. A sock having cushioning material in a recessed achilles configuration is obtained.

### Example 4

A 2-piece from fitting sleeve was made from a fabric containing 57% polyester, 33% nylon, and 10% lycra and was placed over a core pattern. A mixture of molten C-Flex 1970-W5 (67 wt%) and Duoprime 70 mineral of (33 wt%) was poured into a cavity and the core with sleeve was placed in the cave to procedure a cushion liner.

### Example 5

A 2-piece form fitting sleeve was made of a nylon/Lycra material. An adapter was injection molded into the closed end of the sleeve with polypropylene. The sleeve with adapter was then inverted end placed over a core. A mixture of molten C-Flex 1970-W5 (50 wt%) and Duoprime 70 mineral oil (50 wt%) was poured into a cavity and the core with sleeve was placed in the cavity to produce a cushion locking liner. After molding, a pin was threaded into the adapter which was adapted to mate with a lock built into a prosthatic socket.

The invention articles are designed primarily for the human wearer, and thus are sized appropriately. Diameters typically vary from 25.4-203.2 mm (1-8 inchce) (unstretched) and overal lengths typically vary from 25.4- 762 (1 - 30 inches) (unstretched). Obviously, numerous modifications are available which fall within the scope of the invention and appended claims.

## Claims

1. A cushion liner, for enclosing an amputation stump, said liner comprising a fabric covering having an open end for introduction of said stump and a closed end opposite said open end, wherein said fabric is coated on at least the inside thereof with a form-fitting polymeric cushioning material which contacts the skin of the amputation stump when worn by a user to minimise or eliminate air pockets, **characterized in that** the textile material used to make said fabric has a thickness of from 0.635 mm to 3.175 mm (0.025 inches to 0.125 inches).

2. A cushion liner as claimed in claim 1 wherein said polymeric cushioning material has a thickness profile such that the polymeric cushioning material is thicker at a closed end of the liner than at an open end.

3. A cushion liner as claimed in any preceding claim, wherein said liner has an uneven distribution of said polymeric cushioning material, said uneven distribution comprising a thinner posterior middle and upper portion, and a thicker distal anterior-medial and anterior-lateral portion.

4. A cushion liner as claimed in any preceding claim, wherein said polymeric cushioning material is arranged in a recessed achilles configuration.

5. A cushion liner as claimed in any preceding claim, having a length from closed end to open end of from 254-635 mm (10-25 inches).

6. A cushion liner as claimed in claim 1, wherein said fabric covering is coated on only the inside thereof with said polymeric cushioning material.

7. A cushion liner as claimed in claim 1, wherein said fabric covering is coated with the polymeric cushioning material by dipping the fabric in liquefied or molten polymeric material.

8. A cushion liner as claimed in any preceding claim wherein said polymeric cushioning material includes a biocide.

9. A cushion liner as claimed in any preceding claim wherein said polymeric cushioning material includes a vitamin.

10. A cushion liner as claimed in any preceding claim wherein said liner can be donned by inverting and rolling onto the amputation stump.

11. A cushion liner as claimed in any preceding claim further comprising docking means at the closed end thereof.

12. A cushion liner as claimed in claim 11 wherein said docking means is molded to the exterior of said liner.

13. A cushion liner as claimed in claim 11 wherein said docking means is molded to said fabric on the exterior of said liner.

14. A cushion liner as claimed in any preceding claim, wherein the polymeric cushioning material is selected from the group consisting of foamed or non-foamed thermoplastic elastomers, thermoplastic rubbers, silicons rubbers and polymeric gel compositions comprising a block copolymer.

15. A cushion liner as claimed in any one of claims 1 to 14 wherein said polymeric cushioning material comprises a block copolymer and mineral oil.

16. A cushion liner as claimed in claim 15 wherein said polymeric cushioning material has a Shore A durometer of 1-20.

17. A cushion liner as claimed in claim 15 or claim 16 wherein said polymeric cushioning material comprises 5-85% by weight mineral oil.

18. A cushion liner as claimed in any one of claims 15 to 17 wherein said block copolymer is selected from the group consisting of a styrene-isoprene/butadiene block copolymer, a styrene-ethylene/propylene block copolymer, or a styrene-ethylene/butadiene-styrene block copolymer.

19. A cushion liner as claimed in any preceding claim having a range of distal radius of 19-102 mm (3/4"-4").

## Patentansprüche

1. Polstervorrichtung zum Einschließen eines Amputationsstumpfes, wobei die Vorrichtung eine Stoffbedeckung umfasst, die ein offenes Ende zum Einführen des Stumpfes und ein dem offenen Ende gegenüberliegendes geschlossenes Ende aufweist, wobei der Stoff auf wenigstens dessen Innenseite mit einem eng anliegenden Polymer-Polstermaterial beschichtet ist, welches die Haut des Amputationsstumpfes kontaktiert, wenn durch einen Verwender getragen, um Lufttaschen zu minimieren oder zu eliminieren, **dadurch gekennzeichnet, dass** das Textilmaterial, das zur Herstellung des Stoffes verwendet wird, eine Dicke von 0,635 mm bis 3,175 mm (0,025 Inch bis 0,125 Inch) besitzt.

2. Polstervorrichtung gemäß Anspruch 1, wobei das Polymer-Polstermaterial ein solches Dickenprofil aufweist, dass das Polymer-Polstermaterial an einem geschlossenen Ende der Vorrichtung dicker als an einem offenen Ende ist.

3. Polstervorrichtung gemäß einem vorhergehenden Anspruch, wobei die Vorrichtung eine ungleichförmige Verteilung des Polymer-Polstermaterials besitzt, wobei die ungleichförmige Verteilung einen dünneren hinteren Mittel- und Oberteil und einen dickeren entfernten Vordermittel- und Vorderseitenteil umfasst.

4. Polstervorrichtung gemäß einem vorhergehenden Anspruch, wobei das Polymer-Polstermaterial in einem vertieften Achilles-Aufbau angeordnet ist.

5. Polstervorrichtung gemäß einem vorhergehenden Anspruch, die eine Länge vom geschlossenen Ende zum offenen Ende von 254 - 635 mm (10 - 25 Inch) besitzt.

6. Polstervorrichtung gemäß Anspruch 1, wobei die Stoffbedeckung nur auf deren Innenseite mit dem Polymer-Polstermaterial beschichtet ist.

7. Polstervorrichtung gemäß Anspruch 1, wobei die Stoffbedeckung durch Eintauchen des Stoffes in verflüssigtes oder geschmolzenes Polymermaterial mit dem Polymer-Polstermaterial beschichtet ist.

8. Polstervorrichtung gemäß einem vorhergehenden Anspruch, wobei das Polymer-Polstermaterial ein Biozid einschließt.

9. Polstervorrichtung gemäß einem vorhergehenden Anspruch, wobei das Polymerpolstermaterial ein Vitamin einschließt.

10. Polstervorrichtung gemäß einem vorhergehenden Anspruch, wobei die Vorrichtung durch Invertieren und Aufrollen auf den Amputationsstumpf angezogen werden kann.

11. Polstervorrichtung gemäß einem vorhergehenden Anspruch, die ferner eine Andockeinrichtung an deren geschlossenem Ende umfasst.

12. Polstervorrichtung gemäß Anspruch 11, wobei die Andockeinrichtung an die Außenseite der Vorrichtung geformt ist.

13. Polstervorrichtung gemäß Anspruch 11, wobei die Andockeinrichtung an den Stoff auf der Außenseite der Vorrichtung geformt ist.

14. Polstervorrichtung gemäß einem vorhergehenden Anspruch, wobei das Polymer-Polstermaterial aus der Gruppe ausgewählt ist, die aus geschäumten oder nicht geschäumten thermoplastischen Elastomeren, thermoplastischen Kautschuken, Silikonkautschuken und Polymer-Gelzusammensetzungen, die ein Blockcopolymer umfassen, besteht.

15. Polstervorrichtung gemäß einem der Ansprüche 1 bis 14, wobei das Polymer-Polstermaterial ein Blockcopolymer und Mineralöl umfasst.

16. Polstervorrichtung gemäß Anspruch 15, wobei das Polymer-Polstermaterial eine Shore-A-Härte von 1-20 besitzt.

17. Polstervorrichtung gemäß Anspruch 15 oder Anspruch 16, wobei das Polymer-Polstermaterial 5-85 Gew.-% Mineralöl umfasst.

18. Polstervorrichtung gemäß einem der Ansprüche 15-17, wobei das Blockcopolymer aus der Gruppe ausgewählt ist, die aus einem Styrol-Isopren/Butadien-Blockcopolymer, einem Styrol-Ethylen/Propylen-Blockcopolymer oder einem Styrol-Ethylen/Butadien-Styrol-Blockcopolymer besteht.

19. Polstervorrichtung gemäß einem vorhergehenden Anspruch, die einen Bereich des entfernten Radius von 19 - 102 mm (3/4"-4") besitzt.

## Revendications

1. Garniture de matelassage pour confiner un moignon d'amputation, ladite garniture comprenant un tissu couvrant comportant une extrémité ouverte pour introduction dudit moignon et une extrémité fermée opposée à ladite extrémité ouverte, ledit tissu étant recouvert sur au moins sa partie intérieure d'un matériau polymère de matelassage épousant une forme qui est en contact avec la peau du moignon d'amputation lorsque la garniture est portée par un utilisateur dans le but de minimiser ou d'éliminer des poches d'air, **caractérisée en ce que** la matière textile utilisée pour fabriquer ledit tissu présente une épaisseur de 0,635mm à 3,175 mm (0,025 pouce à 0,125 pouce).

2. Garniture de matelassage selon la revendication 1, dans laquelle ledit matériau polymère de matelassage a un profil d'épaisseur tel que le matériau polymère de matelassage est plus épais au niveau d'une extrémité fermée de la garniture qu'au niveau d'une extrémité ouverte.

3. Garniture de matelassage selon l'une quelconque des revendications précédentes, dans laquelle ladite garniture a une répartition irrégulière dudit matériau polymère de matelassage, ladite répartition irrégulière comprenant une partie postérieure intermédiaire et supérieure plus mince, et une partie antérieure médiale et antérieure latérale distale plus épaisse.

4. Garniture de matelassage selon l'une quelconque des revendications précédentes, dans laquelle ledit matériau polymère de matelassage est agencé en une configuration de tendon d'Achille en retrait.

5. Garniture de matelassage selon l'une quelconque des revendications précédentes, ayant une longueur d'une extrémité fermée à une extrémité ouverte de 254 à 635 mm (10 à 25 pouces).

6. Garniture de matelassage selon la revendication 1, dans laquelle ledit tissu couvrant n'est revêtu que sur sa partie intérieure dudit matériau polymère de matelassage.

7. Garniture de matelassage selon la revendication 1, dans laquelle ledit tissu couvrant est revêtu du matériau polymère de matelassage par immersion directe du tissu dans un matériau polymère liquéfié ou fondu.

8. Garniture de matelassage selon l'une quelconque des revendications précédentes, dans laquelle ledit matériau polymère de matelassage inclut un biocide.

9. Garniture de matelassage selon l'une quelconque des revendications précédentes, dans laquelle ledit matériau polymère de matelassage inclut une vitamine.

10. Garniture de matelassage selon l'une quelconque des revendications précédentes, dans laquelle ladite garniture peut être appliquée par inversion et roulement sur le moignon d'amputation.

11. Garniture de matelassage selon l'une quelconque des revendications précédentes, comprenant en outre un moyen de fixation au niveau de son extrémité fermée.

12. Garniture de matelassage selon la revendication 11, dans laquelle ledit moyen de fixation est moulé sur la partie extérieure de ladite garniture.

13. Garniture de matelassage selon la revendication 11, dans laquelle ledit moyen de fixation est moulé sur ledit tissu sur la partie extérieure de ladite garniture.

14. Garniture de matelassage selon l'une quelconque des revendications précédentes, dans laquelle le matériau polymère de matelassage est choisi à partir du groupe constitué d'élastomères thermoplastiques expansés ou non expansés, de caoutchoucs thermoplastiques, de caoutchoucs silicones et de compositions de gel polymère comprenant un copolymère en masse.

15. Garniture de matelassage selon l'une quelconque des revendications 1 à 14, dans laquelle ledit matériau polymère de matelassage comprend un copolymère en masse et une huile minérale.

16. Garniture de matelassage selon la revendication 15, dans laquelle ledit matériau polymère de matelassage a une dureté Shore A de 1 à 20.

17. Garniture de matelassage selon la revendication 15 ou la revendication 16, dans laquelle ledit matériau polymère de matelassage comprend 5 à 85 % en poids d'huile minérale.

18. Garniture de matelassage selon l'une quelconque des revendications 15 à 17, dans laquelle ledit copolymère en masse est choisi à partir du groupe constitué d'un copolymère en masse de styrène-isoprène/butadiène, d'un copolymère en masse de styrène-éthylène/propylène, ou d'un copolymère en masse de styrène-éthylène/butadiène-styrène.

19. Garniture de matelassage selon l'une quelconque des revendication précédentes, ayant une plage de rayon distal de 19 à 102 mm (3/9" à 9").
